# EUROPEAN PATENT SPECIFICATION

(11) **EP 0 891 368 B1**
(45) Date of publication and mention of the grant of the patent: **09.08.2000**
(21) Application number: 96910707.7
(22) Date of filing: 02.04.1996
(51) Int. Cl.: C07F 13/00, C07F 15/04, C07F 15/02, C07F 5/00, C07C 407/00, C07C 27/00

(54) **CATALYTIC PRODUCTION OR ARYL ALKYL HYDROPEROXIDES BY POLYNUCLEAR TRANSITION METAL AGGREGATES**
KATALYTISCHE HERSTELLUNG VON ARYL-ALKYLHYDROPEROXIDEN UNTER VERWENDUNG VON MEHRKERNIGEN ÜBERGANGSMETALLAGREGATEN
PRODUCTION CATALYTIQUE D'ARYLALKYL-HYDROPEROXYDES PAR DES AGREGATS POLYNYUCLEAIRES DE METAUX DE TRANSITION

(43) Date of publication of application: 20.01.1999
(73) Proprietor: EXXON CHEMICAL PATENTS INC., Baytown, TX 77520-2149 (US); EXXON RESEARCH AND ENGINEERING COMPANY, Florham Park, New Jersey 07932-0390 (US)
(72) Inventor: BOND, Jeffrey, E., Flemington, NJ 08822 (US); GORUN, Sergio, M., Providence DI02906 (US); SCHRIVER, George, W., Somerville, NJ 08876 (US); STIBRANY, Robert, T., Long Valley, NJ 07853 (US); VANDERSPURT, Thomas, H., Delaware Township, NJ 08559 (US); VIA, Grayson, H., Westfield, NJ 07090 (US); ZHANG, Baoshan, Upton, NY 11973 (US); DAKKA, Jihad, M., B-3010 Kessel-lo (BE)
(74) Representative: White, Nicholas John, Dr.
(86) International application number: US9604522
(87) International publication number: WO9736910

(56) References cited:
- WO-A-94/08932
- US-A- 5 099 045
- JOURNAL OF THE AMERICAN CHEMICAL SOCIETY, vol. 118, no. 1, 1996, pages 65-69, XP002017901 GRUSH, M.M. ET AL.: "MANGANES L-EDGE X-RAY ABSORPTION SPECTROSCOPY OF MANGANESE CATALASE FROM LACTOBACILLUS PLANTARUM AND MIXED VALENCE MANGANESE COMPLEXES"
- CHEMICAL ABSTRACTS, vol. 125, Columbus, Ohio, US; abstract no. 167562, TAKAI, T. ET AL.: "PREPARATION OF ARYLALKYL HYDROPEROXIDES" XP002017902 & JP,A,08 169 874 (MITSUI PETROCHEMICAL IND.) 2 July 1996

## Description

### FIELD OF INVENTION

This invention relates to a new class of compounds. More specifically, it relates to compounds having an oxo (hydroxo) bridged tetranuclear, mixed metal core and to their method of preparation and use in catalysing the oxidation of aryl alkyl hydrocarbons in the presence of an oxygen-containing gas to produce aryl alkyl hydroperoxides.

### BACKGROUND OF THE INVENTION

For information on the plethora of known oxo bridged tetranuclear metal compounds, reference is made to the following review articles: K. Wieghardt, *Angew. Chem.lnt. Ed. English,* 28, p. 1153 (1989); J, B. Vincent, G. Christou, Adv. *Inorg. Chem.,* 33, p. 197, (1989); G. Christou, *Acc. Chem. Res*., 22, p.328 (1989); and G.W. Brudvig, R.H. Crabtree, *Prog. Inorg. Chem.,* 37, p. 99 (1989). These articles describe primarily manganese complexes in which the manganese is present in various oxidation states, spatial arrangements and the like. Other literature references and tetranuclear manganese complexes are referred to and disclosed in US Patent 5, 025, 101.

compounds having an oxo (hydroxo) bridged tetranuclear, mixed metal core, however, have not been previously reported. Hence, the ability to prepare such materials is highly desired.

The production of organic hydroperoxides from aryl alkyl hydrocarbons in the presence of various transition metal salt complexes has been described in the literature. See, for example, US Patent 2,954,405 disclosing the production of organic hydroperoxides by autoxidation of hydrocarbons in the presence of molecular oxygen and metal phthalocyanines as catalysts. Similarly, US Patent 4,013,725 discloses a process for preparing hydroperoxides in a homogeneous system by autoxidizing secondary alkyl group-substituted methylbenzenes in the presence of water, a base, an oxygen containing gas, and a watersoluble chelate compound in which multidentate ligands are coordinated to at least one metal from the class of cobalt, nickel, manganese, copper, and iron.

In US Patent 5,025,101 and US Patent 5,183,945 tetranuclear manganese complexes, and their method of preparation, and their use as catalyst in the production of hydroperoxide are disclosed.

In part the present invention is predicated on the discovery that certain compounds having an oxo (hydroxo) bridged tetranuclear, mixed metal core, are useful as oxidation catalysts, in the presence of oxygen containing gas, for the production of organic hydroperoxides.

### SUMMARY OF THE INVENTION

Accordingly, in one embodiment, the present invention comprises a composition of matter having the formula:

M[Mₐ(M_{b})₃(O)ₓ(OH)_{y}(O₂CR)₂L₂]

wherein M is an ammonium ion, one or more alkali metal ions, one or more alkaline earth metal ions, or mixtures thereof. M may thus be a single ion or a mixture of ions of differing valence; Ma is a divalent metal or mixture of divalent metals, Mb is trivalent metal or mixture of trivalent metals; x and y are numerical values the sum of which equals 2. The exact number of ions M and their valence will depend on the value of y and x and the consequential negative valence (n-) of the complex anion [Mₐ(M_{b})3(O)ₓ(OH)_{y}(O2CR)2L2]ⁿ⁻. When y=x=1 the negative valence of the complex anion will be 4- and M may therefore be four ammonium or alkali metal ions or mixtures thereof, or may be two alkaline earth metal ions or may be two ammonium or alkali metal ions or mixtures thereof and one alkaline earth metal ion. When y=2 and x=0 the negative valence of the complex anion is 3-, when y=0 and x=2 the negative valence of the complex anion is 5-; and there are various combinations of cations which may be used to form an electrically neutral material with such valences. R is hydrogen or a hydrocarbyl group; and L is a ligand having the formula:

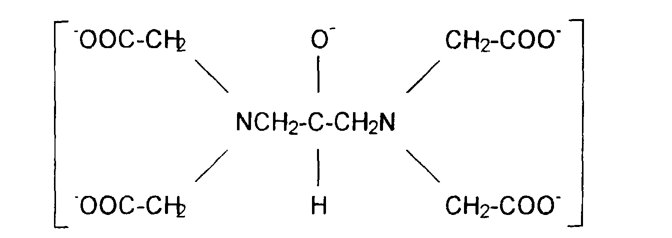

In a further embodiment the present invention comprises a method of preparing compounds having the formula:

M[Mₐ(M_{b})₃(O)ₓ(OH)_{y}(O₂CR)₂L₂]

wherein M, L, Mₐ, M_{b}, x , y and R are as listed above. The method comprises forming an aqueous solution containing: (a) a compound having the formula: 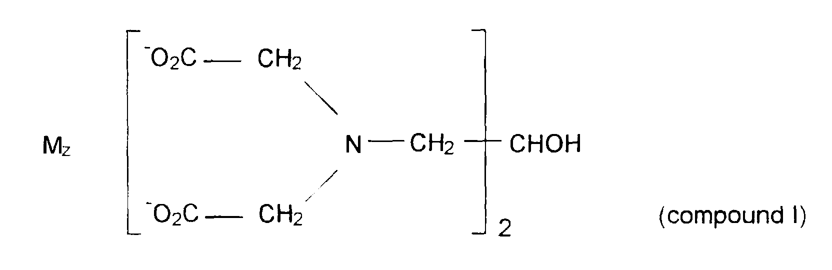 wherein M consists of ammonium, alkaline, or an alkaline earth metal ions or mixtures thereof in sufficient amounts for compound I to be electrically neutral, (b) a mixture of a water soluble salt of at least one divalent metal Mₐ and a trivalent metal M_{b}; and (c) a source of a carboxylate, RCO₂-, where R is hydrogen or a hydrocarbyl group whereby a compound having the formula M [Mₐ(M_{b})₃(O)ₓ(OH)_{y}(O₂CR)L₂] is prepared. In one embodiment, the solution is formed in situ by adding an oxidant, such as air, oxygen or hydrogen peroxide to an aqueous solution of compound I, a carboxylate source and (i) a water soluble salt of a divalent metal Ma and a metal oxidizable to a trivalent metal, M_{b}, or (ii) a water soluble salt of a divalent metal oxidizable to a trivalent metal, M_{b}, the addition being in an amount and for a time sufficient to oxidize at least part of oxidizable divalent metal to the trivalent metal M_{b}.

The compounds of the present invention, in which at least one of Mₐ and M_{b} is selected from a metal have an incomplete d electronic shell, having particular suitability for use in oxidation of hydrocarbons as peroxide decomposers. Other compounds of the present invention form solid solutions with the former thereby serving as a diluent and catalyst support for the former compounds.

The present invention further provides a method for preparing organic hydroperoxides, which method comprises contacting an aryl alkyl hydrocarbon having a benzylic hydrogen with an oxygen containing gas in the presence of an oxo (hydroxo) bridged tetranuclear metal complex having a mixed metal core, one metal of the core being a divalent metal selected from Zn, Cu, Fe, Co, Ni, Mn or mixtures thereof and another metal being a trivalent metal selected from In, Fe, Mn, Ga and Al.

### BRIEF DESCRIPTION OF THE DRAWING

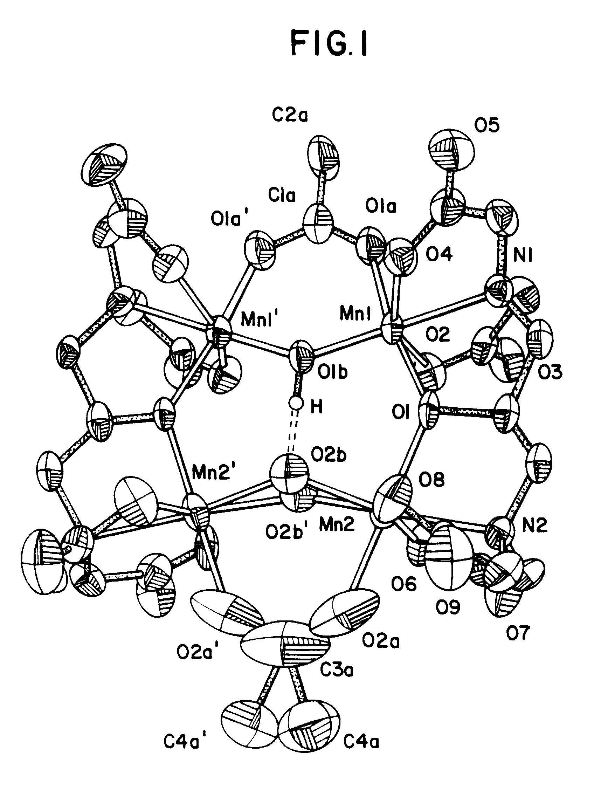

Figure 1 in the instant application illustrates the oxo (hydroxo) bridged tetranuclear metal core for the composition

M[Mₐ(M_{b})₃(O)ₓ(OH)_{y}(O₂CR)₂L₂]

in which x=y=1, M_{b}=Mn and Mₐ= a mixture of Mn and Cu.

Figures 2 and 3 show the weight percent of cumene hydroperoxide produced from cumene in the presence of and various catalysts by the oxidation process of the present invention. Also shown in Figure 3 is the amount of cumene hydroperoxide produced in the presence of cumene, air and cumene hydroperoxide as initiator.

### DETAILED DESCRIPTION OF THE INVENTION

The compounds of the present invention have the formula:

M[Mₐ(M_{b})₃(O)ₓ(OH)_{y}(O₂CR)₂L₂]

wherein M is an ammonium ion, one or more alkali metal ions, one or more alkaline earth metal ions, or mixtures thereof, preferably Li, Na, K, NH₄, Mg, Ca, Sr, Ba or mixtures thereof. R is hydrogen or a hydrocarbyl group especially alkyl, aryl, and aralkyl groups. Preferably R is an alkyl group having from 1 to about 30 carbon atoms and , more preferably, R has from 1 to about 10 carbon atoms; and when R is an aralkyl group, it preferably has from 7 to 10 carbon atoms. Mₐ is a divalent metal like Zn, Cu, Fe, Co, Ni, Mn; and, M_{b} is a trivalent metal like In, Fe, Mn, Ga, Al. The subscripts x and y are numerical values the sum of which is 2; and L is a ligand having the formula:

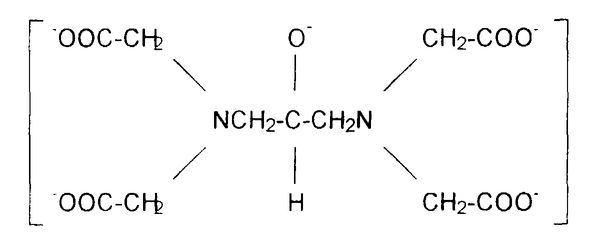

As shown in the accompanying figure, these novel compounds have a core structure of four metal atoms which are bridged by oxo and hydroxo groups and, hence, these compounds are referred to as oxo(hydroxo) bridged tetranuclear metal compounds.

In the process for the preparation of compounds of the present invention exemplary salts of Mₐ and M_{b} include metal chlorides, bromides, nitrates, tetrafluoroborates, and sulfates, provided, however, that sulfates are not used when M is Ca, Ba or Sr. Exemplary sources of carboxylate include carboxylic acids and alkaline metal salts of carboxylic acids. Among suitable aqueous containing solutions are water, water-alcohol, and water-dimethyl fromamide mixtures. In general it is particularly preferred to use water as the solvent.

The molar ratio of compound I to the metal salts containing Mₐ and M_{b} generally will be in the range of from about 1:1 to about 1:3 and preferably about 1:2.

Because the acid analogue of the ligand L is commercially available, it is particularly preferred in the practice of the present invention to prepare an aqueous containing solution of compound I by first neutralizing an aqueous solution of its conjugated acid with an alkali or alkaline earth metal hydroxide or mixture thereof, and thereafter adding the metal carboxylates or metal salts and source of carboxylate.

In one embodiment, the solution is formed in situ by adding an oxidant, such as air, oxygen or hydrogen peroxide to an aqueous solution of compound I, a carboxylate source and (i) a water soluble salt of a divalent metal Mₐ and a metal oxidizable to a trivalent metal, M_{b}., or (ii) a water soluble salt of a divalent metal oxidizable to a trivalent metal, M_{b}, the addition being in an amount and for a time sufficient to oxidize at least part of the oxidizable divalent metal to the trivalent metal M_{b}.

As pointed out above, this aqueous mixture is then oxidized if needed. The need for oxidation arises only when M_{b} is not present in its trivalent state to start with. As shown later in Preparations 11 and 12, for the preparation of the complex in which Mₐ is Mn and M_{b} is Ga, there is no need for oxidation because Ga is already present as Ga(lll). On the other hand, as shown in Preparations 8, 9 and 10 for the preparation of the complex in which Mₐ in Zn and M_{b} is Mn, one may start with Mn(ll) salts and oxidize the Mn(ll) to the required Mn(lll) state. This is achieved by adding an oxidant such as air, molecular oxygen, or hydrogen peroxide. When air or oxygen is employed, the gas is bubbled through the mixture at temperatures in the range of about 20°C to about 60°C and in an amount sufficient to form the desired compound. When hydrogen peroxide is used as the oxidant, in general the peroxide will have a concentration range of about 10 wt % to 30 wt % and, preferably, about 25 wt % and will be used in excess, for example, up to about 10 times the stoichiometric amount required. The addition of hydrogen peroxide to the reaction mixture results in an exothermic reaction and consequently it is particularly preferred to maintain the temperature of the reaction mixture during oxidation in the range of about 10°C to 60°C, and preferably, in the range of about 20°C to 40°C.

Typically, the desired compound is recovered by fractional crystallization from suitable solvents such as water-dimethylformamide mixtures.

The compounds of the present invention have utility as catalysts for the production of aryl alkyl hydroperoxides. For example following the procedure set forth in US Patent 5, 183, 945 which is incorporated herein by reference, the compounds of the invention, in which at least one of Mₐ and M_{b} is a metal having an incomplete d electronic shell, may be used to catalyse the oxidation of cumene to cumene hydroperoxide.

The oxidation process of the present invention is therefor carried out by contacting an aryl alkyl hydrocarbon with an oxygen-containing gas and at least one tertiary alkyl hydroperoxide and a catalytically effective amount of either an oxo (hydroxo) bridged tetranuclear metal complex having a mixed metal core.

The aryl alkyl hydrocarbons employed as starting materials in this process may be obtained from commercial sources. Preferably the aryl alkyl hydrocarbons will have a melting point within the range of temperatures at which the process of the present invention is operated or be capable of being solubilized in an inert solvent. Importantly, the aryl alkyl hydrocarbons should contain a benzylic hydrogen. An example of useful aryl alkyl hydrocarbons is represented by the general formula: 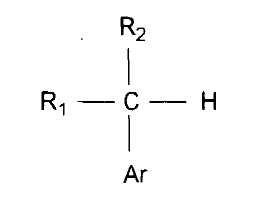 where R₁ and R₂ may be the same or different organo groups, preferably alkyl groups having from 1 to about 10 carbon atoms, or hydrogen and Ar is an aromatic or substituted aromatic group, such as alkyl and halo, substitute aromatic groups. R₁ and R₂ may most preferably be bonded together to form a cycloaliphatic group such as, for example, a cyclohexyl group in which case the aryl alkyl hydrocarbon will be cyclohexylbenzene. In the case of alkyl substituted aromatic groups, the alkyl group generally will have from 1 to about 10 carbon atoms.

The oxygen containing gas used preferably is air or oxygen, and, more preferably, is air.

The amount of catalyst used will vary depending upon the nature and amount of the organic starting material to be oxidized. In general from about 0.001 to about 0.5 parts by weight of catalyst per 100 parts of substrate and preferably from about 0.1 to about 0.2 parts per 100 parts of substrate are satisfactory.

In the process of the present invention, the solvent for the reaction is preferably an excess amount of the aryl alkyl hydrocarbon to be oxidized, e.g. cyclohexyl benzene; however, hydrocarbons such as benzene, chlorobenzene, halogenated hydrocarbons, and the like may be employed as solvents.

Preferably the hydrocarbon, catalyst and oxygen or oxygen containing gas are contacted in such a way as to provide for good mixing, such as rapid bubbling of the gas through the mixture or mechanical agitation. Preferably air is used in the contacting.

The flow rate of air is not critical and the optimum rate will vary depending on the reaction temperature and pressure employed. In the case of the oxidation of cumene to cumene hydroperoxide, for example, the flow rate of air preferably will be at least 2 liters/hr up to about 10 liters/hr per 100 g of cumene.

The reaction temperature may range from about 0°C to about 90°C, preferably from about 60°C to about 80°C. Temperatures at the lower end of the preferred range are more desirable.

Typically the contacting is conducted at atmospheric pressure. Importantly, the present invention results in the selection oxygenation of the aryl alkyl hydrocarbon and does not oxygenate the aromatic or aliphatic hydrocarbons present in the starting material.

In conducting the method of this invention, the formation of the corresponding organic hydroperoxide can be monitored, for example, by analyzing aliquots by NMR, iodometric titration, chromatography or other means readily known to one skilled in the art. Also, the organic hydroperoxide is readily recovered from the reaction mixture by conventional methods, for example distillation, and as a result, the process may be run in batch or continuously. In a continuous process, the aryl alkyl hydrocarbon starting material may be passed over the catalyst in a bed or otherwise contacted with the catalyst. The organic hydroperoxide may be withdrawn and the organic starting material recycled.

The present invention further provides a process for the manufacture of phenol and cyclohexanone from cyclohexyl benzene which process comprises;
(a) contacting cyclohexyl benzene with an oxygen-containing gas and an oxo(hydroxo) bridged tetranuclear metal complex having mixed metal core, under catalytic oxidation conditions to form cyclohexylbenzene hydroperoxide and
(b) decomposing the cyclohexylbenzene hydroperoxide to form a mixture comprising phenol and cyclohexanone.

Preferably the cyclohexylbenzene hydroperoxide is decomposed with acid.

The invention will now further be described by means of the following examples.

### EXAMPLES

### A. Catalyst Preparation:

A series of detailed catalyst preparations are provided herein to illustrate the general preparation techniques previously described. In the preparations which follow, DHPTA refers to 1,3-diamino-2-hydroxypropane-N,N,N'N'-tetraacetic acid; DMF is dimethylformamide; MeOH is methyl alcohol. Also, in those preparations in which the subscript s is used in the formula, s is a value greater than zero but less than 1, depending upon the ratio of mixed divalent metals used.

### Preparation 1

Preparation of Ba₂[(Cu_{0.4}Mn_{0.6})Mn₃(O)(OH)(O₂CCH₃)₂L₂]

In a 50 ml Erlenmeyer flask, 268 mg of DHPTA was added to ~7 ml H₂0. Then enough Ba(OH)₂.H₂0 was added to give a clear solution, followed by 93 mg of Cu(O₂CCH₃)₂.H₂O and 343 mg of Mn(O₂CCH₃)₂.4H₂O. About 1/2 ml of MeOH and ~1 ml of DMF was added next, while stirring. After the pH was brought to ~8 by adding slowly Ba(OH)₂, the solution was treated with ~1 ml of ~25% H₂O₂. During this addition, the solution color changed from blue to brown. This filtered solution gave crystals by evaporation. The structure of the product was determined by simple crystal x-ray diffraction and sorption spectroscopy. The simple crystal x-ray analysis revealed the presence of CU(II) ions which selectively replaced the Mn(ll) ions.

### Preparation 2

Preparation of Ca₂[(CuₛMn₁₋ₛ)Mn₃(O)(OH)(O₂CCH₃)₂L₂]

The procedure of Preparation 1 was followed except that Ca(OH)₂ was used in place of Ba(OH)₂.

### Preparation 3

Preparation of Ba₂[CuFe₃(O)(OH)(O₂CCH₃)₂L₂]

In a 50 ml Erlenmeyer flask, 268 mg of DHPTA and 100 µI of 100% CH₃COOH was added to ~8 ml H₂O. The pH was brought to ~6 with Ba(OH)₂.H₂O. Then 110 mg of Cu(O₂CCH₃)₂.4H₂O and 3 45 mg of Fe(NO₃)₃.6H₂O was added to the solution. Then the pH was brought to ~7.5 with Ba(OH)₂.H₂O and 1 ml DMF was added. The yellow green solution was filtered and the product crystallized by evaporation.

### Preparation 4

Alternate Preparation of Ba₂[CuFe₃(O)(OH)(O₂CCH₃)₂L₂]

In a 50 ml Erlenmeyer flask, 268 mg of DHPTA was added to 10 ml H₂O. The solution was brought to pH ~6 with Ba(OH)₂.H₂O. Then 105 mg of Cu(O₂CCH₃)₂.4H₂O and 540 mg of Fe(C10₄)₃.6H₂O and 100 µl of CH₃COOH were all added. After stirring, the pH was brought to 7.5-8.0 with Ba(OH)₂ and 1 ml DMF was added. The crystalline product was obtained by evaporation.

### Preparation 5

Preparation of Ca₂[ZnGa₃(O)(OH)(O₂CCH₃)₂L₂]

268 mg of DHPTA was added to about 8 ml H₂O then brought to pH 6 using Ca(OH)₂. Then 115 mg of Zn(O₂CCH₃).2H₂O and 3 20 mg of Ga(NO₃)₃.6H₂O and 100 µI glacial acetic acid was added to the solution. The pH was then brought to about 8 with Ca(OH)₂ and 2 ml of DMF was added. The solution was filtered and the product was obtained by crystallization.

### Preparation 6

Preparation of Ba₂[ZnGa₃(O)(OH)(O₂CCH₃)₂L₂]

In a 50 ml Erlenmeyer flask, 268 mg of DHPTA was added to ~8 ml H₂O. The solution was brought to pH 6 with Ba(OH)₂.H₂O. Then 120 mg of Zn(O₂CCH₃)₂.2H₂O and 330 mg of Ga(NO₃)₃.6H₂O was added while stirring. The pH was brought to 7.5 with Ba(OH)₂.H₂O. Following the addition of 1 ml of DMF, the solution was filtered and the product crystallized by evaporation.

### Preparation 7

Preparation of Na₄[ZnGa₃(O)(OH)(O₂CCH₃)₂L₂]

In a 50 ml Erlenmeyer flask 268 mg of DHPTA was added to ~6 ml H₂O. The solution was brought to pH ~6.0 with NaOH. Then three drops of 100% CH₃COOH was added, followed by 105 mg Zn(O₂CCH₃)₂.2H₂O and 340 mg of Ga(NO₃)₃.6H₂O. The pH was then brought to ~8 using NaOH, the solution was filtered and 1 ml DMF added. The product was obtained by crystallization.

### Preparation 8

Preparation of Ca₂[(ZnₛMn₁₋ₛ)Mn₃(O)(OH)(O₂CCH₃)₂L₂]

268 mg of DHPTA was added with 10 ml H₂O to a 50 ml flask. The solution was brought to pH 8 with powdered Ca(OH)₂. In another flask, 330 mg of Mn(O₂CCH₃)₂.4H₂O and 105 mg Zn(O₂CCH₃)₂.2H₂O was dissolved in 10 ml of 1:1 H₂O:MeOH. Then 200 mg of CaCl₂ was also dissolved in the (Zn,Mn)(O₂CCH₃)₂ solution. Next, the Mn/Zn containing solution was added to the DHPTA solution and stirred for 5 minutes. The pH was adjusted to 8.0 with Ca(OH)₂, after which 1/2 ml of 30% H₂O₂ was added dropwise. Finally, 4 ml of DMF was added, the solution was filtered and the product was obtained by crystallization.

### Preparation 9

Alternative preparation of Ca₂[(ZnₛMn₁₋ₛ)Mn₃(O)(OH)(O₂CCH₃)₂L₂]

In a 50 ml Erlenmeyer flask 268 mg of DHPTA was added to ~7 ml H₂O. The solution was brought to pH ~6.5 with Ca(OH)₂. Then 105 mg at Zn(O₂CCH₃)₂.2H₂O and 330 mg of Mn(O₂CCH₃)₂.6H₂O was added to the solution. The pH was brought to ~8 with Ca(OH)₂ and 0.5 ml of ~30% H₂O₂ was added giving off heat and gas. 1 ml of DMF was then added. The crystalline product was obtained by evaporation.

### Preparation 10

Preparation of Ba₂[(ZnₛMn₁₋ₛ)Mn₃(O)(OH)(O₂CCH₃)₂L₂]

In a 50 ml Erlenmeyer flask containing 5 ml H₂O, 100 mg of Ba(OH)₂.H₂O was neutralized with concentrated HC1 to pH 7. Then 330 mg of Mn(O₂CCH₃)₂.4H₂O and 105 mg Zn(O₂CCH₃)₂.2H₂O were added, along with 10 ml of 1:1 H₂O/MeOH. In another 50 ml flask, 268 mg of DHPTA was added to 10 ml of H₂O. This was neutralized with solid Ba(OH)₂ while stirring. The two solutions were mixed together and stirred for about 10 minutes, after which the pH was adjusted to 8.0 with solid Ba(OH)₂.H₂O. Next, 0.5 ml of 30% H₂O₂ was added dropwise. Then 5 ml of DMF was added, the solution was stirred 10 minutes and filtered. The product was obtained by crystallization.

### Preparation 11

Preparation of Na₄[MnGa₃(O)(OH)(O₂CCH₃)₂L₂]

In a 50 ml Erlenmeyer flask, 268 mg of DHPTA was added to ~8 ml H₂O under an Ar blanket. The pH was brought to ~6 with NaOH. Then 100 µI of 100% CH₃COOH was added, followed by 110 mg of Mn(O₂CCH₃)₂.6H₂O and 340 mg of Ga(NO₃)₃.6H₂O. The pH was then brought to 8.5 with NaOH. The pale pink crystalline product was obtained via MeOH diffusion in the aqueous solution.

### Preparation 12

Preparation of K₄[MnGa₃(O)(OH)(O₂CCH₃)₂L₂]

In a 50 ml Erlenmeyer flask, 268 mg of DHPTA was added to ~7 ml H₂O. The solution was brought to pH ~6 with KOH. Then 60 µl of 100% CH₃COOH was added, followed by 100 mg of Mn(O₂CCH₃)₂.6H₂O and 3 50 mg of Ga(NO₃)₃.6H₂O. The pH was brought to ~8 with KOH, the solution filtered and layered with MeOH to give pale pink crystalline product.

### Preparation 13

Preparation of Ba₂[(NiₛMn₁₋ₛ)Mn₃(O)(OH)(O₂CCH₃)₂L₂]

In a 50 ml Erlenmeyer flask, 268 mg of DHPTA was added to ~8 ml H₂O. The pH was brought to ~6.5 with Ba(OH₂).H₂O. Next, 110 mg of Ni(O₂CCH₃)₂.6H₂O and 330 mg of Mn(O₂CCH₃)₂.6H₂O were added. The pH was brought to ~8 and then 1 ml H₂O₂ (30%) and 1 ml DMF were added slowly. The crystalline product was obtained by evaporation from the filtered solution.

### Preparation 14

Preparation of Ba₂[FeGa₃(O)(OH)(O₂CCH₃)₂L₂]

In a 50 ml Erlenmeyer flask, 268 mg DHPTA was added to ~7 ml H₂O containing 100 µl of 100% CH₃COOH and the pH was brought to ~6.5 with Ba(OH)₂.H₂O. Then 454 mg of Ga(NO₃)₂.6H₂O was added, pH was brought to 8 with Ba(OH)₂.H₂O, the solution filtered under argon and Fe(O₂CCH₃)₂ was added while stirring. Crystals formed via methanol diffusion.

### Preparation 15

Preparation of K₄[CoMn₃(O)(OH)(O₂CCH₃)₂L₂]

In a 50 ml Erlenmeyer flask, 268 mg of DHPTA was added to ~10 ml H₂O and the pH was brought to about 6.5 with KOH. Then 110 mg of CO(O₂CCH₃)₂.4H₂O and 320 mg of Mn(O₂CCH₃)₂.6H₂O was added to the solution. The pH was brought to ~8 with KOH and ~2 ml of 30% H₂O₂ was added dropwise giving a dark red-brown solution. Then 2 ml of DMF was added and the solution was filtered. Crystals were obtained by evaporation.

### Preparation 16

Preparation of Ca₂[Fe₄(O)(OH)(O₂CCH₃)₂L₂]

In a 50 ml Erlenmeyer flask, 268 mg of DHPTA was added to ~8 ml H₂O and the pH was adjusted to ~7.5 with Ca(OH)₂. Then 420 mg of Fe(O₂CCH₃)₂ was added.. Then 1 ml of ~30% H₂O₂ was added giving a dark green solution. The pH was readjusted to 8.0 with Ca(OH)₂. Following the addition of 1 ml DMF, the solution was filtered and crystallized by evaporation.

### Examples 1 to 5

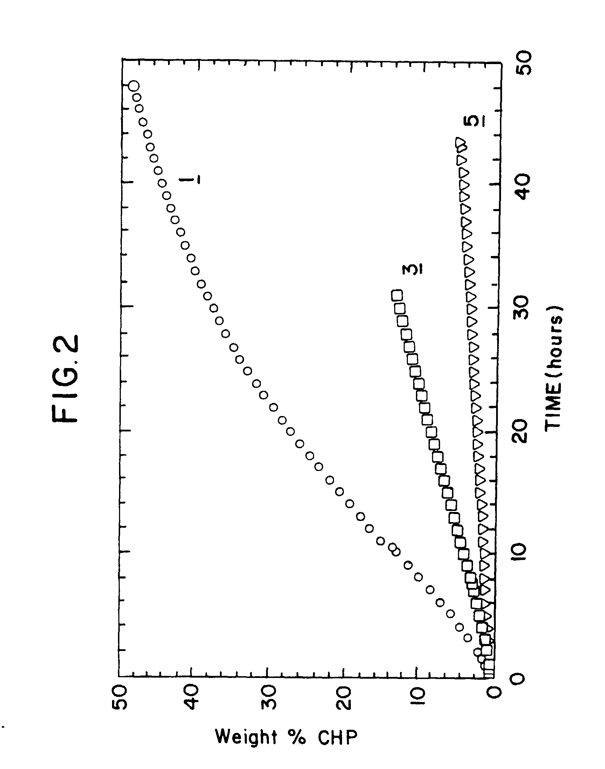

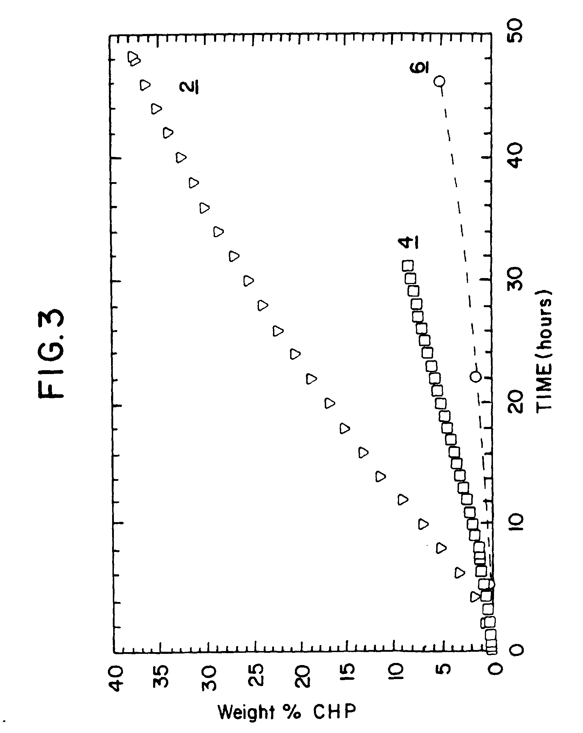

A series of runs were conducted using as catalysts the compounds listed in Table 1 below. In each example, 0.1g of solid catalyst was added to 50g of neat cumene in a flask. Air at 1 atm of pressure, ws bubbled through the reaction mixture at a rate of 30ml/min. The temperature of the reaction is maintained at 65°C. The conversion of cumene to cumene hydroperoxide (CHP) was monitored by iodometric titrations and oxygen uptake. The accompanying Figures 2 and 3 show the weight% CHP produced vs. time for various catalysts labelled as in Table 1. In Figure 3, line 6 corresponds to the uncatalyzed auto-oxidation of cumene described in the Comparative Example 1.

**Table 1**

| Example | M [Mₐ(M_{b})₃(O)ₓ(OH)_{y}(O₂CR)₂L₂] | | |
|---|---|---|---|
| | M | Ma | Mb |
| 1 | Ba | NiₛMn₁₋ₛ | Mn |
| 2 | K | Mn | Ga |
| 3 | Na | Zn | Ga |
| 4 | Ba | Cu | Fe |
| 5 | Ba | Fe | Ga |

### Comparative Example 1

12.0 grams of cumene (0.1 mole) was combined with 0.2 cm³ of cumene hydroperoxide (as an initiator) and heated to 100°C in a oxygen atmosphere with vigorous stirring. In three hours about 3% oxidation to cumene hydroperoxide occurs. Thus the rate observed for the CHP auto catalysed oxidation at 100°C is about 1% per hour. To compare this with an oxidation carried out at 65°C a simple extrapolation can be done using the Arrhenius equation. Such as extrapolation indicates a reaction rate of about 0.1% per hour at 65°C which is one order of magnitude smaller than that obtained with the catalysts of this invention.

## Claims

1. A composition of matter having the formula:
M[Mₐ(M_{b})₃(O)ₓ(OH)_{y}(O₂CR)₂L₂]
wherein M is an ammonium ion, one or more alkali metal ions, one or more alkaline earth metal ions, or mixtures thereof; Ma is a divalent metal or mixture of divalent metals, Mb is trivalent metal or mixture of trivalent metals; x and y are numerical values the sum of which equals 2, R is hydrogen or a hydrocarbyl group; and L is a ligand having the formula:

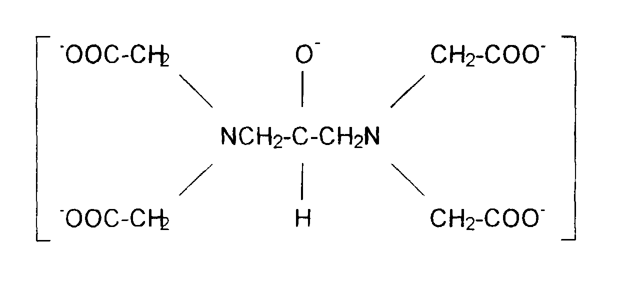

2. The composition of claim 1 wherein the divalent metal Mₐ or mixture thereof is selected from Zn, Mn, Fe, Cu, Ni, and Co.

3. The composition as claimed in claim 2 wherein the trivalent metal M_{b} or a mixture thereof, is selected from Mn, Fe, Ga, Al, and In.

4. A method of preparing a compound of formula:
M[Mₐ(M_{b})₃(O)ₓ(OH)_{y}(O₂CR)₂L₂]
wherein M is an ammonium ion, one or more alkali metal ions, one or more alkaline earth metal ions, or mixtures thereof; Mₐ is a divalent metal or mixture of divalent metals, M_{b} is trivalent metal or mixture of trivalent metals; x and y are numerical values the sum of which equals 2, R is hydrogen or a hydrocarbyl group; and L is a ligand having the formula: 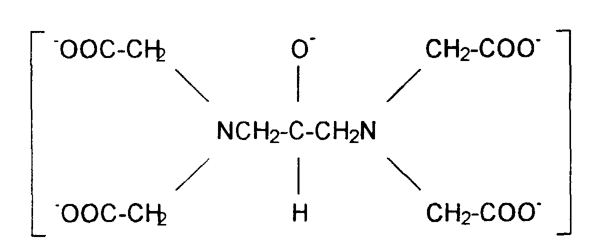 the method comprising forming an aqueous containing solution of: (a) a first compound of the formula: 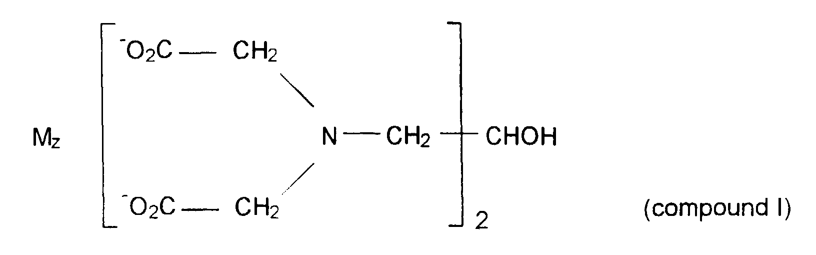 wherein M consists of ammonium, alkaline, or an alkaline earth metal ions or mixtures thereof in sufficient amounts for compound I to be electrically neutral, (b) a mixture of a water soluble salt of at least one divalent metal Mₐ and a trivalent metal M_{b}; and (c) a source of a carboxylate, RCO₂-, where R is hydrogen or a hydrocarbyl group whereby a compound having the formula M [Mₐ(M_{b})₃(O)ₓ(OH)_{y}(O₂CR)₂L₂] is prepared.

5. A method as claimed in claim 4 wherein the mixture of water soluble salts is formed in situ by adding an oxidant, such as air, oxygen or hydrogen peroxide to an aqueous solution of the first compound, a carboxylate source and (i) a water soluble salt of at least one divalent metal Mₐ and a metal oxidizable to a trivalent metal, M_{b}, or (ii) a water soluble salt of at least one divalent metal oxidizable to a trivalent metal, M_{b}, the addition of oxidant being in an amount and for a time and at a temperature sufficient to oxidize at least part of oxidizable divalent metal to the trivalent metal M_{b}.

6. A method for oxidizing an aryl alkyl hydrocarbon to an organic hydroperoxide comprising:
contacting an aryl alkyl hydrocarbon having a benzylic hydrogen with an oxygen containing gas and a catalytically effective amount of an oxo(hydroxo) bridged tetranuclear metal complex having a mixed metal core, one metal of the core being a divalent metal selected from Zn, Cu, Fe, Co, Ni, Mn or mixtures thereof and another metal being a trivalent metal selected from In, Fe, Mn, Ga and Al or mixtures thereof.

7. The method of claim 6 wherein the aryl alkyl hydrocarbon has the general formula: 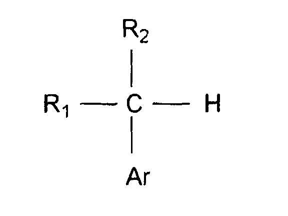 when R₁ and R₂ are independently hydrogen or organo groups and Ar is an aromatic group.

8. The method of claim 7 wherein the organo groups are alkyl groups of from 1 to about 10 carbon atoms.

9. The method of claim 6 wherein the oxygen-containing gas is air.

10. The method of claim 6 wherein the temperature is in the range of from about 0°C to about 90°C.

11. The method of claim 10 wherein the aryl alkyl hydrocarbon is a liquid at the temperature range.

12. The method of claim 6 wherein the aryl alkyl hydrocarbon is dissolved in an inert solvent.

13. The method of claim 6 wherein the aryl alkyl hydrocarbon is cyclohexylbenzene and the resultant hydroperoxide is cyclohexyl benzene hydroperoxide.

14. A process for the manufacture of phenol and cyclohexanone from cyclohexyl benzene which process comprises;
(a) contacting cyclohexyl benzene with an oxygen-containing gas and an oxo(hydroxo) bridged tetranuclear metal complex having mixed metal core, under catalytic oxidation conditions to form cyclohexylbenzene hydroperoxide and
(b) decomposing the cyclohexylbenzene hydroperoxide to form a mixture comprising phenol and cyclohexanone.

## Patentansprüche

1. Stoffzusammensetzung mit der Formel:
M[Mₐ(M_{b})₃(O)ₓ(OH)_{y}(O₂CR)₂L₂]
in der M Ammoniumion, ein oder mehrere Alkalimetallionen, ein oder mehrere Erdalkalimetallionen oder Mischungen derselben ist, Mₐ ein zweiwertiges Metall oder eine Mischung von zweiwertigen Metallen ist, M_{b} dreiwertiges Metall oder eine Mischung von dreiwertigen Metallen ist, x und y Zahlenwerte sind, deren Summe 2 ergibt, R Wasserstoff oder eine Kohlenwasserstoffgruppe ist und L ein Ligand mit der Formel: 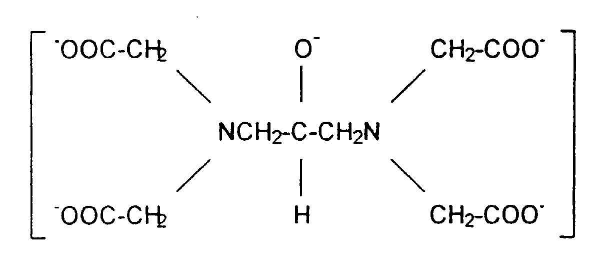 ist.

2. Zusammensetzung nach Anspruch 1, wobei das zweiwertige Metall Mₐ oder die Mischung desselben ausgewählt ist aus Zn, Mn, Fe, Cu, Ni und Co.

3. Zusammensetzung nach Anspruch 2, wobei das dreiwertige Metall M_{b} oder eine Mischung desselben ausgewählt ist aus Mn, Fe, Ga, Al und In.

4. Verfahren zur Herstellung einer Verbindung mit der Formel:
M[Mₐ(M_{b})₃(O)ₓ(OH)_{y}(O₂CR)₂L₂]
in der M Ammoniumion, ein oder mehrere Alkalimetallionen, ein oder mehrere Erdalkalimetallionen oder Mischungen derselben ist, Mₐ ein zweiwertiges Metall oder eine Mischung von zweiwertigen Metallen ist, M_{b} dreiwertiges Metall oder eine Mischung von dreiwertigen Metallen ist, x und y Zahlenwerte sind, deren Summe 2 ergibt, R Wasserstoff oder eine Kohlenwasserstoffgruppe ist und L ein Ligand mit der Formel: 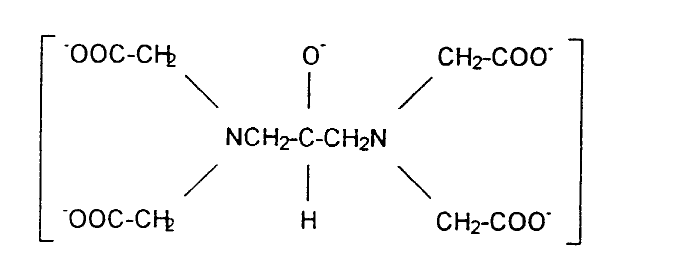 ist, wobei das Verfahren die Bildung einer wässrigen Lösung von: (a) einer ersten Verbindung mit der Formel: 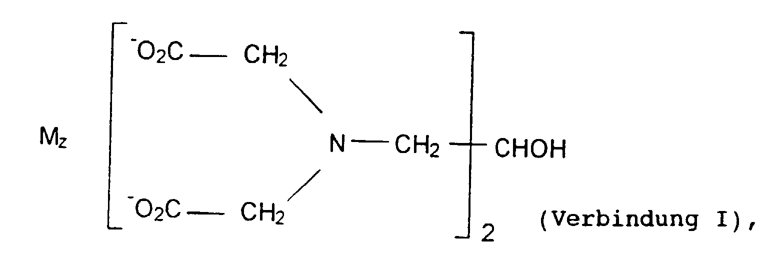 in der M aus Ammonium-, Alkali- oder Erdalkalimetallionen oder Mischungen derselben in ausreichenden Mengen besteht, so dass Verbindung I elektrisch neutral ist, (b) einer Mischung eines wasserlöslichen Salzes von mindestens einem zweiwertigen Metall Mₐ und einem dreiwertigen Metall M_{b} und (c) einer Quelle von Carboxylat, RCO₂⁻, wobei R Wasserstoff oder eine Kohlenwasserstoffgruppe ist, umfasst, wodurch eine Verbindung der Formel M[Mₐ(M_{b})₃(O)ₓ(OH)_{y}(O₂CR)₂L₂] hergestellt wird.

5. Verfahren nach Anspruch 4, wobei die Mischung der wasserlöslichen Salze in situ gebildet wird, indem ein Oxidationsmittel wie Luft, Sauerstoff oder Wasserstoffperoxid zu einer wässrigen Lösung der ersten Verbindung, einer Carboxylatquelle und (i) einem wasserlöslichen Salz von mindestens einem zweiwertigen Metall Mₐ und einem zu einem dreiwertigen Metall M_{b} oxidierbaren Metall oder (ii) einem wasserlöslichen Salz von mindestens einem zweiwertigen Metall, das zu einem dreiwertigen Metall M_{b} oxidierbar ist, wobei die Zugabe des Oxidationsmittels in einer Menge und für eine Zeitdauer und bei einer Temperatur erfolgt, die ausreichend sind, um mindestens einen Teil des oxidierbaren zweiwertigen Metalls zu dem dreiwertigen Metall M_{b} zu oxidieren, gegeben wird.

6. Verfahren zum Oxidieren eines Arylalkylkohlenwasserstoffes zu einem organischen Hydroperoxid, bei dem:
ein Arylalkylkohlenwasserstoff mit benzylischem Wasserstoff mit Sauerstoff enthaltendem Gas und einer katalytisch wirksamen Menge eines oxo(hydroxo)-verbrückten vierkernigen Metallkomplexes, der einen gemischten Metallkern aufweist, wobei ein Metall des Kerns ein zweiwertiges Metall ist, das aus Zn, Cu, Fe, Co, Ni, Mn oder Mischungen derselben ausgewählt ist, und ein weiteres Metall ein dreiwertiges Metall ist, das aus In, Fe, Mn, Ga und Al oder Mischungen derselben ausgewählt ist, kontaktiert wird.

7. Verfahren nach Anspruch 6, wobei der Arylalkylkohlenwasserstoff die allgemeine Formel: 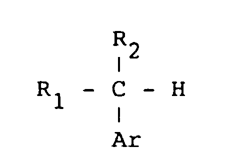 aufweist, wobei R₁ und R₂ unabhängig Wasserstoff oder Organogruppen sind und Ar eine aromatische Gruppe ist.

8. Verfahren nach Anspruch 7, wobei die Organogruppen Alkylgruppen mit einem bis etwa 10 Kohlenstoffatomen sind.

9. Verfahren nach Anspruch 6, wobei das Sauerstoff enthaltende Gas Luft ist.

10. Verfahren nach Anspruch 6, wobei die Temperatur im Bereich von etwa 0 °C bis etwa 90 °C liegt.

11. Verfahren nach Anspruch 10, wobei der Arylalkylkohlenwasserstoff bei dem Temperaturbereich flüssig ist.

12. Verfahren nach Anspruch 6, wobei der Arylalkylwasserstoff in inertem Lösungsmittel gelöst wird.

13. Verfahren nach Anspruch 6, wobei der Arylalkylkohlenwasserstoff Cyclohexylbenzol ist und das resultierende Hydroperoxid Cyclohexylbenzolhydroperoxid ist.

14. Verfahren zur Herstellung von Phenol und Cyclohexanon aus Cyclohexylbenzol, bei dem
(a) Cyclohexylbenzol mit Sauerstoff enthaltendem Gas und einem oxo(hydroxo)-verbrücktem vierkernigen Metallkomplex mit gemischtem Metallkern unter katalytischen Oxidationsbedingungen kontaktiert wird, um Cyclohexylbenzolhydroperoxid zu bilden und
(b) das Cyclohexylbenzolhydroperoxid zersetzt wird, um eine Mischung zu bilden, die Phenol und Cyclohexanon umfasst.

## Revendications

1. Composition répondant à la formule :
M [Mₐ(M_{b})₃(O)ₓ(OH)_{y}(O₂CR)₂L₂]
dans laquelle M représente un ion ammonium, un ou plusieurs ions de métaux alcalins, un ou plusieurs ions de métaux alcaline-terreux ou un de leurs mélanges ; Mₐ représente un métal divalent ou un mélange de métaux divalents, M_{b} représente un métal trivalent ou un mélange de métaux trivalents ; x et y sont des valeurs numériques dont la somme est égale à 2, R représente l'hydrogène ou un groupe hydrocarbyle ; et L représente un ligand répondant à la formule :

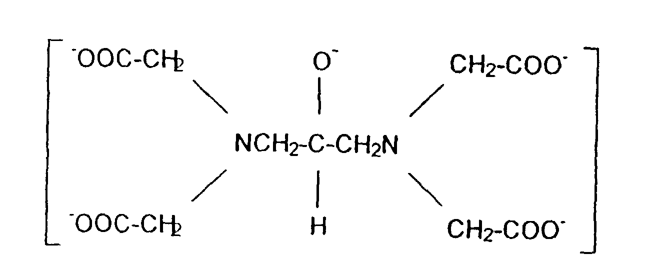

2. Composition suivant la revendication 1, dans laquelle le métal divalent Mₐ ou le mélange de tels métaux divalents est choisi entre Zn, Mn, Fe, Cu, Ni et Co.

3. Composition suivant la revendication 2, dans laquelle le métal trivalent M_{b} ou un mélange de métaux trivalents M_{b} est choisi entre Mn, Fe, Ga, Al et In.

4. Procédé pour la préparation d'un composé de formule :
M[Mₐ(M_{b})₃(O)ₓ(OH)_{y}(O₂CR)₂L₂]
dans laquelle M représente un ion ammonium, un ou plusieurs ions de métaux alcalins, un ou plusieurs ions de métaux alcalino-terreux ou un de leurs mélanges ; Mₐ représente un métal divalent ou un mélange de métaux divalents, M_{b} représente un métal trivalent ou un mélange de métaux trivalents ; x et y sont des valeurs numériques dont la somme est égale à 2, R représente l'hydrogène ou un groupe hydrocarbyle ; et L représente un ligand répondant à la formule : 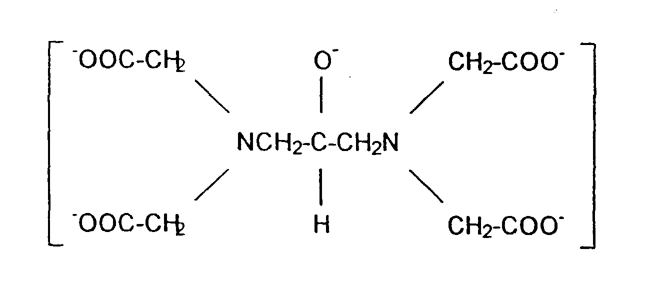 procédé qui comprend la formation d'une solution aqueuse : (a) d'un premier composé de formule : 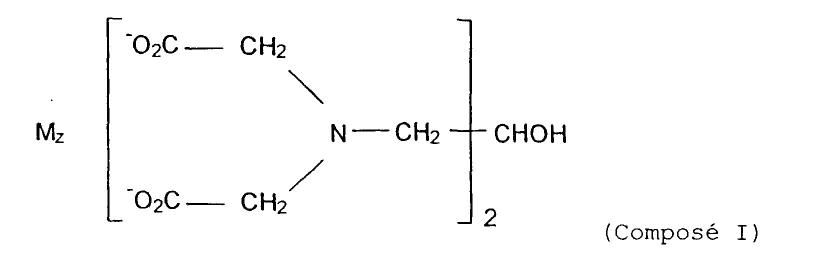 dans laquelle M consiste en un ion ammonium, un ion de métal alcalin ou un ion de métal alcalino-terreux ou un de leurs mélanges en des quantités suffisantes pour que le composé I soit électriquement neutre, (b) d'un mélange d'un sel hydrosoluble d'au moins un métal divalent Mₐ et d'un métal trivalent M_{b} ; et (c) d'une source d'un carboxylate, RCO₂⁻, dans lequel R représente l'hydrogène ou un groupe hydrocarbyle, ce qui permet de préparer un composé répondant à la formule M[Mₐ(M_{b})₃(O)ₓ(OH)_{y}(O₂CR)₂L₂].

5. Procédé suivant la revendication 4, dans lequel le mélange de sels hydrosolubles est formé in situ en ajoutant un oxydant, tel que l'air, l'oxygène ou le peroxyde d'hydrogène à une solution aqueuse du premier composé, d'une source de carboxylate et (i) d'un sel hydrosoluble d'au moins un métal divalent Mₐ et d'un métal oxydable en un métal trivalent, M_{b}, ou (ii) d'un sel hydrosoluble d'au moins un métal divalent oxydable en un métal trivalent, M_{b}, l'addition de l'oxydant étant effectuée en une quantité, pendant un temps et à une température suffisants pour oxyder au moins une partie du métal divalent oxydable en le métal trivalent M_{b}.

6. Procédé pour oxyder un arylalkylhydrocarbure en un hydroperoxyde organique, comprenant :
la mise en contact d'un arylalkylhydrocarbure ayant un atome d'hydrogène benzylique avec un gaz contenant de l'oxygène et une quantité catalytiquement efficace d'un complexe métallique tétracyclique à pontage oxo(hydroxo) ayant un noyau métallique mixte, un métal du noyau étant un métal divalent choisi entre Zn, Cu, Fe, Co, Ni, Mn et leurs mélanges et un autre métal étant un métal trivalent choisi entre In, Fe, Mn, Ga, Al et leurs mélanges.

7. Procédé suivant la revendication 6, dans lequel l'arylalkylhydrocarbure répond à la formule générale : 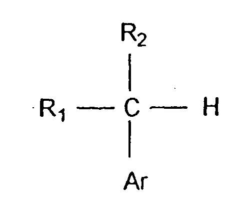 dans laquelle R₁ et R₂ représentent, indépendamment, l'hydrogène ou des groupes organiques et Ar représente un groupe aromatique.

8. Procédé suivant la revendication 7, dans lequel les groupes organiques sont des groupes alkyle ayant 1 à environ 10 atomes de carbone.

9. Procédé suivant la revendication 6, dans lequel le gaz contenant de l'oxygène est l'air.

10. Procédé suivant la revendication 6, dans lequel la température est comprise dans l'intervalle d'environ 0°C à environ 90°C.

11. Procédé suivant la revendication 10, dans lequel l'arylalkylhydrocarbure est un liquide dans cette plage de températures.

12. Procédé suivant la revendication 6, dans lequel l'arylalkylhydrocarbure est dissous dans un solvant inerte.

13. Procédé suivant la revendication 6, dans lequel l'arylalkylhydrocarbure est le cyclohexylbenzène et l'hydroperoxyde résultant est l'hydroperoxyde de cyclohexylbenzène.

14. Procédé pour la production de phénol et de cyclohexanone à partir de cyclohexylbenzène, procédé qui comprend :
(a) la mise en contact de cyclohexylbenzène avec un gaz contenant de l'oxygène et un complexe métallique tétracyclique à pontage oxo(hydroxo) ayant un noyau métallique mixte, dans des conditions d'oxydation catalytique pour former un hydroperoxyde de cyclohexylbenzène, et
(b) à décomposer l'hydroperoxyde de cyclohexylbenzène pour former un mélange comprenant du phénol et de la cyclohexanone.
